(19) Europäisches Patentamt
European Patent Office
Office européen des brevets

(11) EP 0 701 821 A1

(12) **EUROPEAN PATENT APPLICATION**

(43) Date of publication:
**20.03.1996 Bulletin 1996/12**

(51) Int. Cl.[6]: **A61L 2/18**, A01N 47/44

(21) Application number: **95114496.3**

(22) Date of filing: **15.09.1995**

(84) Designated Contracting States:
**DE FR GB**

(30) Priority: **19.09.1994 JP 223553/94**

(71) Applicant: **TOMEY TECHNOLOGY CORPORATION**
**Nagoya-shi, Aichi-ken (JP)**

(72) Inventors:
- **Sugiura, Makoto,**
**c/o Tomey Technology Corporation**
**Nagoya-shi, Aichi-ken (JP)**
- **Ibaraki, Keiko,**
**c/o Tomey Technology Corporation**
**Nagoya-shi, Aichi-ken (JP)**
- **Tanaka, Shinji,**
**c/o Tomey Technology Corporation**
**Nagoya-shi, Aichi-ken (JP)**

(74) Representative: **Türk, Gille, Hrabal, Leifert**
**Brucknerstrasse 20**
**D-40593 Düsseldorf (DE)**

(54) **Solution for contact lenses**

(57) A solution for contact lenses comprising at least one of a biguanide derivative represented by the general formula (I):

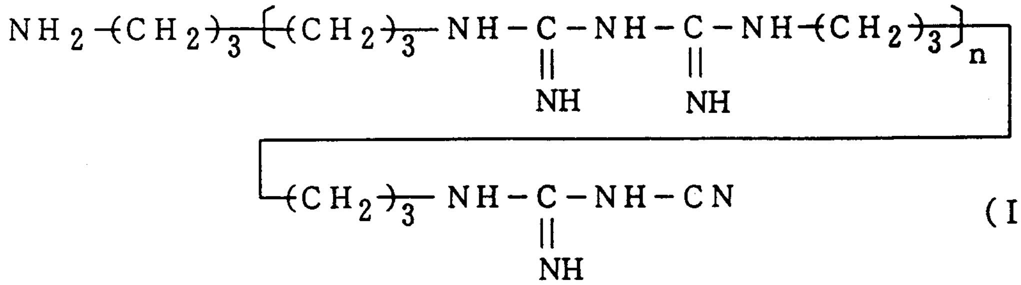

(I)

wherein n is an integer of 1 to 500 and a salt of the biguanide derivative in an effective amount for antimicrobial activity, and at least one of phosphoric acid and a phosphoric acid salt in an amount of 80 to 250 mM. The solution for contact lenses can be suitably used for disinfection, rinse and storage of a contact lens, and is not adsorbed to and concentrated in a contact lens.

EP 0 701 821 A1

## Description

The present invention relates to a solution for contact lenses, and more particularly to a solution for contact lenses, which can be suitably used for disinfection, storage and rinse of a contact lens.

Currently, there have been, in general, widely used a hydrogel contact lens (water-absorptive soft contact lens) which is excellent in sensation of wearing, a hard contact lens which has high oxygen permeability and the like.

Any of these contact lenses necessitate care such as cleaning after wearing in eyes. Particularly, a hydrogel contact lens necessitates disinfection before wearing in eyes.

As a disinfecting method for the above hydrogel contact lens, there are a heat disinfecting method comprising boiling a soft contact lens in a treating solution for a contact lens and a non-heat disinfecting method comprising disinfecting a contact lens with hydrogen peroxide or a disinfectant, which is so-called a cold disinfecting method.

However, in any of these disinfecting methods, operation and handling thereof are complicated. Particularly, when hydrogen peroxide is used in the above non-heat disinfecting method, its operation becomes complicated because a neutralizing treatment is necessitated. Also, when a disinfectant is used in the non-heat disinfecting method, a side effect such as allergy sometimes occurs.

Among the above non-heat disinfecting methods in which a disinfectant is used, taking notice that allergy is apt to occur when the disinfectant permeates the inside of a contact lens, in order to prevent the disinfectant from permeating the inside of a contact lens, a solution for disinfecting and storing a contact lens, which contains biguanide as a disinfectant has been proposed (Japanese Unexamined Patent Publication No. 18043/1974 and Japanese Unexamined Patent Publication No. 85301/1986), and the solution has been widely used.

However, the above solution for disinfecting and storing a contact lens, which contains biguanide is not sufficient for safety for eyes because the biguanide is sometimes adsorbed to a contact lens in a high concentration depending on the kinds of the contact lens used.

The present invention has been accomplished in consideration of the above prior art.

An object of the present invention is to provide a solution for contact lenses, which can be suitably used for disinfection, rinse and storage of a contact lens, and is not adsorbed to and concentrated in a contact lens.

This and other objects of the present invention will become apparent from the description hereinafter.

In accordance with the present invention, there is provided a solution for contact lenses comprising at least one of a biguanide derivative represented by the general formula (I):

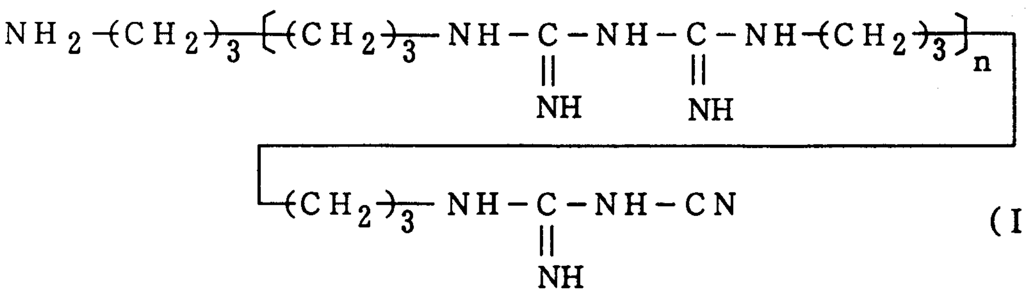

(I)

wherein n is an integer of 1 to 500 and a salt of the biguanide derivative in an effective amount for antimicrobial activity, and at least one of phosphoric acid and a phosphoric acid salt in an amount of 80 to 250 mM.

The solution for contact lenses of the present invention comprises, as mentioned above, at least one of a biguanide derivative represented by the general formula (I):

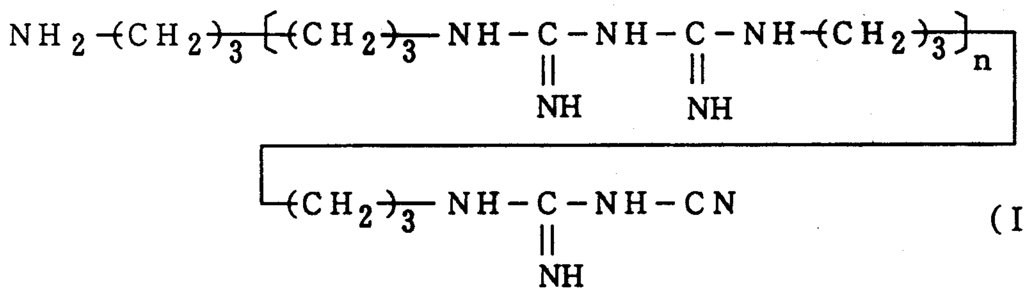

(I)

wherein n is an integer of 1 to 500 and a salt of the biguanide derivative (hereinafter referred to as "biguanide derivative (salt)") in an effective amount for antimicrobial activity, and at least one of phosphoric acid and a phosphoric acid salt (hereinafter referred to as "phosphoric acid (salt)") in an amount of 80 to 250 mM.

The biguanide derivative represented by the general formula (I) and the salt of the biguanide derivative, which are used in the present invention, have biguanide group represented by the formula:

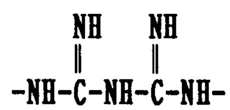

,

which is composed of two of a guanidine group represented by the formula:

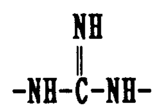

,

and have great antimicrobial spectrum.

In the concept of the above biguanide derivative, there are included hexamethylenebiguanide, a hexamethylenebiguanide polymer, and a salt of the hexamethylenebiguanide or hexamethylenebiguanide polymer and a basic compound.

As typical commodities of the biguanide derivative and the salt of the biguanide derivative, there can be cited, for instance, commodities commercially available from Zeneca under the trade name of Arlagard E, Cosmocil CQ, Proxel IB and Vantocil IB, commodities commercially available from Brooks under the trade name of Mikrokill and Mikrokill 20, and the like.

As one of the examples of the biguanide derivative and the salt of the biguanide derivative, there can be cited, for instance, 20 % aqueous solution of hydrochloric acid salt of polyhexamethylenebiguanidine commercially available from Zeneca under the trade name of Cosmocil CQ (n in the general formula (I) is 12 or so on the avarage), and the like.

The amount of the biguanide derivative (salt) cannot be indiscriminately decided because the amount varies depending on mycetoma used and the like. For instance, when *Escherichia coli* which is Gram-negative bacteria is used as organism, the minimum inhibitory concentration of the above biguanide derivative (salt) is 20 ppm. When *Pseudomonas aeruginosa* which is Gram-negative bacteria is used, the minimum inhibitory concentration is 100 ppm. When *Staphylococcus aureus* which is Gram-positive bacteria is used, the minimum inhibitory concentration is 20 ppm. The biguanide derivative (salt) shows excellent disinfecting properties and bacteriostatics within the above minimum inhibitory concentration. Accordingly, in the present invention, in order that sufficient disinfecting properties and bacteriostatics are exhibited, the amount of the above biguanide derivative (salt) in the solution for contact lenses is adjusted to an effective amount for antimicrobial activity. In order to give the solution for contact lenses sufficient disinfecting properties and

bacteriostatics, it is usually desired that the effective amount for antimicrobial activity is at least 0.1 ppm, preferably at least 0.5 ppm, more preferably at least 0.8 ppm. Also, in order to prevent the biguanide derivative (salt) from adsorbing to a contact lens, it is usually desired that the effective amount for antimicrobial activity is at most 10 ppm, preferably at most 5 ppm, more preferably at most 3 ppm.

Moreover, the biguanide derivative (salt) is, as mentioned above, at least one of the biguanide derivative and the salt of the biguanide derivative.

In the present invention, there is one of great characteristics in that the phosphoric acid (salt) is contained in the solution for contact lenses.

When the above phosphoric acid (salt) and the above biguanide derivative (salt) are used together, unexpectedly, there is exhibited such effect that the biguanide derivative (salt) is prevented from adsorbing to a contact lens.

Moreover, the phosphoric acid (salt) is, as mentioned above, at least one of phosphoric acid and the phosphoric acid salt.

In the concept of the above phosphoric acid, there are included metaphosphoric acid and polyphosphoric acid besides phosphoric acid (orthophosphoric acid, $H_3PO_4$). These can he usually used alone or in an admixture thereof.

As typical examples of the phosphoric acid salt, there can be cited, for instance, sodium dihydrogen phosphate, disodium hydrogen phosphate, potassium dihydrogen phosphate, dipotassium hydrogen phosphate and the like. These can be usually used alone or in an admixture thereof.

Among the above phosphoric acid (salt), sodium dihydrogen phosphate, disodium hydrogen phosphate, potassium dihydrogen phosphate and dipotassium hydrogen phosphate can be suitably used in the present invention because these are, in general, widely used and can be easily obtained.

In order to sufficiently prevent the biguanide derivative (salt) from adsorbing to a contact lens, it is desired that the amount of the phosphoric acid (salt) in the solution for contact lenses is at least 80 mM, preferably at least 95 mM, more preferably at least 100mM. Also, in order to adjust osmotic pressure of the obtained solution for contact lenses to at most physiological osmotic pressure, it is desired that the amount of the phosphoric acid (salt) in the solution for contact lenses is at most 250 mM, preferably at most 200 mM, more preferably at most 150 mM.

In the present invention, in the case that the solution for contact lenses is used as a detergent by improving its cleaning property for a contact lens, it is desired that the solution for contact lenses contains a nonionic surface active agent.

If a cationic surface active agent is contained in the solution for contact lenses as a surface active agent, an active group of the cationic surface active agent is adsorbed to a contact lens and eyes are sometimes stimulated when the obtained solution for contact lenses enters eyes. If an anionic surface active agent is contained in the solution for contact lenses as a surface active agent, a salt is generated by the reaction of the anionic surface active agent with the above biguanide derivative (salt), and precipitated. If an amphoteric surface active agent is contained in the solution for contact lenses as a surface active agent, eyes are stimulated when the solution for contact lenses enters eyes. Accordingly, it is not desired that these surface active agents are contained in the solution for contact lenses.

As typical examples of the above nonionic surface active agent, there can be cited, for instance, polyoxyethylene alkyl ether, polyoxyethylene alkylphenyl ether, polyoxyethylene polyoxypropylene alkyl ether, polyoxyethylene glycerine fatty acid ester, polyoxyethylene sorbitan fatty acid ester, polyethylene glycol fatty acid ester, polyglyceryl fatty acid ester, sorbitan fatty acid ester, propylene glycol fatty acid ester, polyoxyethylene fatty acid amide, polyoxyethylene alkylamine and the like. These can be used alone or in an admixture thereof.

The above nonionic surface active agents are soluble in the solution for contact lenses and show excellent cleaning properties for a contact lens. Among them, there can be particularly suitably used a nonionic surface active agent having an oxypropylene chain as a main chain and an oxyethylene chain at both ends of the oxypropylene chain in the present invention because the above nonionic surface active agent does not impart stimulation to eye tissues and shows high safety.

As typical examples of the above nonionic surface active agent having an oxypropylene chain as a main chain and an oxyethylene chain at both ends of the oxypropylene chain, there can be cited, for instance, Pluronic L-64, Pluronic P-84, Pluronic F-68, Pluronic F-88, Pluronic F-108, Synpelonic PE/F127 and the like.

In order to impart sufficient cleaning properties to an obtained solution for contact lenses, it is usually desired that the content of the nonionic surface active agent in the solution for contact lenses is at least 0.1 w/v %, preferably at least 0.25 w/v %, more preferably at least 0.5 w/v %. Also, in order to improve solubility of the nonionic surface active agent in the solution for contact lenses, it is usually desired that the content of the nonionic surface active agent in the solution for contact lenses is at most 10 w/v %, preferably at most 5 w/v %, more preferably at most 1 w/v %.

Moreover, in order to chelate a polyvalent metal ion contained in a lacrimal fluid, for instance, a sequestering agent such as ethylenediaminetetraacetic acid salt such as sodium ethylenediaminetetraacetate may be added to the solution for contact lenses of the present invention. Also, in order to carry out isotonization of the solution for contact lenses, for instance, sodium chloride and the like may be added to the solution for contact lenses, and osmotic pressure of the solution for contact lenses may be adjusted to 270 to 320 mmol/kg.

When the solution for contact lenses of the present invention is prepared, special methods are not necessitated. The solution for contact lenses can be prepared by dissolving each of components in sterile purified water in a fixed amount, in the same manner as in the preparation of usual aqueous solutions.

The obtained solution for contact lenses is clear. As occasion demands, the solution for contact lenses can be subjected to sterile filtration and the like.

A contact lens can be stored and disinfected by immersing it in the above solution for contact lenses.

Also, a contact lens can be rinsed, cleaned up and disinfected by rinsing or rubbing for washing it with fingers with the above solution for contact lenses.

The solution for contact lenses of the present invention can effectively disinfect a contact lens, and is not adsorbed to and concentrated in a contact lens. Accordingly, the solution for contact lenses shows such effect that harmful influence is not given to eye tissues and a contact lens.

Therefore, according to a treating method for a contact lens by using the solution for contact lenses of the present invention, a contact lens can be suitably disinfected, rinsed and stored.

The solution for contact lenses of the present invention is more specifically described and explained by means of the following Examples. It is to be understood that the present invention is not limited to the Examples, and various changes and modifications may be made in the invention without departing from the spirit and scope thereof.

Example 1

In 50 mℓ of water was dissolved 500 mg of Poloxsamer 407 commercially available from BASF Japan under the trade name of Lutrol F-127 as a nonionic surface active agent to give an aqueous solution of Poloxsamer 407.

Next, in 40 mℓ of water were dissolved 2600 mg of disodium hydrogen phosphate dodecahydrate, 400 mg of sodium dihydrogen phosphate dihydrate, 80 mg of sodium ethylenediaminetetraacetate and 230 mg of sodium chloride, and thereto was added the total amount of the above aqueous solution of Poloxsamer 407 with stirring. Then, thereto was added 0.02 mg of 20 % aqueous solution of hydrochloric acid salt of polyhexamethylenebiguanidine commercially available from Zeneca under the trade name of Cosmocil CQ as a biguanide derivative (salt). Then, purified water was added thereto, so that the total amount thereof was adjusted to 100 mℓ. They were sufficiently stirred to give a solution for contact lenses (concentration of phosphoric acid (salt): 100 mM, osmotic pressure: 295 mmol/kg, pH 7.2).

The obtained solution for contact lenses was subjected to sterile filtration.

Example 2

In Example 1, the same procedure as in Example 1 was repeated except that the amount of disodium hydrogen phosphate dodecahydrate was changed from 2600 mg to 3640 mg and the amount of sodium dihydrogen phosphate dihydrate was changed from 400 mg to 560 mg to give a solution for contact lenses (concentration of phosphoric acid (salt): 150 mM, osmotic pressure: 295 mmol/kg, pH 7.2).

The obtained solution for contact lenses was subjected to sterile filtration.

The solutions for contact lenses obtained in Examples 1 and 2 were subjected to the tests concerning (1) bacteriocidal activity and (2) adsorption of biguanide derivative (salt) to a material for a contact lens, and (3) observation of safety by measuring growth inhibition rate, (4) test for cleaning effect and (5) observation of rinse and storage properties, in accordance with the following methods.

(1) Bacteriocidal activity

Test organisms were prepared in an amount of $10^7$ to $10^8$ cfu/mℓ. To 10 mℓ of each of the solutions for contact lenses was added 0.05 mℓ of each of the test organisms, and the amount of each of the test organisms in the solution was adjusted to $10^5$ to $10^6$ cfu/mℓ.

As the test organisms, *Escherichia coli* (ATCC8739), *Pseudomonas aeruginosa* (ATCC9027) and *Staphylococcus aureus* (ATCC6538) were used.

Next, each of the solutions for contact lenses to which was added each of the above test organisms was collected after a fixed time passed, and disinfecting effect of the solution for contact lenses was examined by measuring vialbe cell count in the solution. The result is shown in Table 1.

(2) Adsorption of biguanide derivative (salt) to a material for a contact lens

In 65 mℓ of each of the solutions for contact lenses was immersed 1 g of a material for a high water-absorptive soft contact lens having a water absorption of 72 % by weight, which was obtained by using dimethyl methacrylamide and N-vinylpyrrolidone as main components, at 35°C for 24 hours. The concentration of residual biguanide derivative (salt) in each of the solutions for contact lenses was measured. Next, each of the solutions for contact lenses, in which had

been immersed the above material for a contact lens, was changed to 65 mℓ of a newly prepared solution for contact lenses, which was composed of the same components as the above, and in the newly prepared solution for contact lenses was immersed the above material for a contact lens at 35°C for 24 hours. The concentration of residual biguanide derivative (salt) was measured. The above procedure was repeated 4 times.

Next, the concentration (adsorbed amount) of the biguanide derivative (salt) in the material for a contact lens was obtained by the following equation.

[Adsorbed amount of the biguanide derivative (salt) in the material for a contact lens] = [(Concentration of the biguanide derivative (salt) in the solution before immersion) - (Concentration of the biguanide derivative (salt) in the solution after immersion)] × [Amount of the solution ] / [(Weight of the material for a high water-absorptive soft contact lens) × (Water absorption of the material for a high water-absorptive soft contact lens)]

The result is shown in Table 2.

(3) Observation of safety by measuring growth inhibition rate

Safety of the solution for contact lenses was evaluated in accordance with cytotoxicity.

Using a plate for cell culture having 24 holes, in 12 holes of them was sown $1 \times 10^5$ cells/mℓ of mouse fibroblast L-929, and it was recognized that the mouse fibroblast adsorbed to the plate after culture for 2 to 3 hours. Then, 100 μℓ and 200 μℓ of the solution for contact lenses were added to each 3 holes of the 12 holes, respectively, to give Sample. Also, 100 μℓ and 200 μℓ of phosphate buffer were added to each the other 3 holes of the 12 holes, respectively, to give Negative Control. After that, as to this plate, culture was carried out for 3 days. Then, the amount (absorbance at 650 nm) of the living cell, i.e. protein was measured in accordance with the Lowry Method (Lowry, O.H., Rosebrough, N.J., Farr, A.L. and Randoll, R.J. (1951), J. Biol. Chem., <u>193</u>, p.265). The growth inhibition rate in the mouse fibroblast was calculated according to the following equation.

[Growth inhibition rate (%)] = [(Absorbance of Negative Control) - (Absorbance of Sample)] × 100 / [(Absorbance of Negative Control)]

The result is shown in Table 3.

(4) Test for cleaning effect

With 97.5 w/v % of saline was mixed 2.5 w/v % of an artificial lipid liquid prepared by dissolving 6 w/v % of sorbitan monooleic acid ester commercially available from Wako Pure Chemical Industries, Ltd. under the trade name of Span 80, 16 w/v % of castor oil, 35 w/v % of lanolin, 5 w/v % of oleic acid, 4 w/v % of sorbitan torioleic acid ester commercially available from Wako Pure Chemical Industries, Ltd. under the trade name of Span 85, 2 w/v % of cetyl alcohol, 2 w/v % of cholesterol and 30 w/v % of cholesterol acetate and homogenizing them with stirring, to give an artificial lipid liquid.

To a contact lens made of the same material for a high water-absorptive soft contact lens having a water absorption of 72 % by weight as used in the above item (2) was dropped 5 μℓ of the above artificial lipid liquid to give an artificial lipidic deposit lens.

The obtained artificial lipidic deposit lens was put on a palm. Then, 3 drops of the solution for contact lenses or saline were dropped to each side of the lenses (6 drops in total), and each of the lenses was cleaned up for 5 seconds. After that, cleaning condition was observed. The result is shown in Table 4.

(5) Observation of rinse and storage properties

After in eyes of a subject was worn a contact lens made of the same material for a high water-absorptive soft contact lens having a water absorption of 72 % by weight as used in the above item (2) for 8 hours, 3 drops of the solution for contact lenses were dropped to one side of the contact lens and the one side was cleaned up 15 times, and the other side of the contact lens was cleaned up in the same manner as in the one side. Then, the contact lens was rinsed with rubbing with adding dropwise the solution for contact lenses thereto for 20 seconds and the contact lens was disinfected by immersing in the solution for contact lenses for at least 4 hours. Next, each of the contact lenses was stored in the solution for contact lenses or saline at 35 ± 2 °C for 7 days. Then, there was examined existence of microorganisms on each surface of the contact lenses and in the solution for contact lenses or saline. The result is shown in Table 5.

<u>Comparative Example 1</u>

In Example 1, the same procedure as in Example 1 was repeated except that the amount of sodium chloride was changed from 230 mg to 490 mg, and 640 mg of boric acid and 120 mg of sodium borate were used instead of 2600

mg of disodium hydrogen phosphate dodecahydrate and 400 mg of sodium dihydrogen phosphate dihydrate to give 100 mℓ of a solution for contact lenses (osmotic pressure: 295 mmol/kg, pH 7.2).

The obtained solution for contact lenses was subjected to sterile filtration, and each of the above items (1) to (3) was evaluated in the same manner as in Examples 1 and 2. The results are shown in Tables 1 to 3.

Comparative Example 2

In Example 1, the same procedure as in Example 1 was repeated except that the amount of sodium chloride was changed from 230 mg to 490 mg, the amount of disodium hydrogen phosphate dodecahydrate was changed from 2600 mg to 1300 mg and the amount of sodium dihydrogen phosphate dihydrate was changed from 400 mg to 200 mg to give 100 mℓ of a solution for contact lenses (concentration of phosphoric acid (salt): 50 mM, osmotic pressure: 295 mmol/kg, pH 7.2).

The obtained solution for contact lenses was subjected to sterile filtration, and the above item (2) was evaluated in the same manner as in Examples 1 and 2. The result is shown in Table 2.

The result of the item (1) Bacteriocidal activity is as shown in Table 1.

Table 1

| Example No. | Test organisms | Beginning (cfu/mℓ) | Survival number (cfu/mℓ) | | |
|---|---|---|---|---|---|
| | | | After 30 minutes | After 1 hour | After 4 hours |
| 1 | *E. coli* | $2.7 \times 10^5$ | $2.9 \times 10^2$ | 0 | 0 |
| | *P. aeruginosa* | $2.9 \times 10^5$ | 16 | 0 | 0 |
| | *S. aureus* | $7.1 \times 10^5$ | $1.6 \times 10^3$ | $2.0 \times 10^2$ | 30 |
| 2 | *E. coli* | $1.5 \times 10^5$ | $2.0 \times 10^2$ | 0 | 0 |
| | *P. aeruginosa* | $1.8 \times 10^5$ | 12 | 0 | 0 |
| | *S. aureus* | $2.7 \times 10^5$ | $8.2 \times 10^2$ | $1.1 \times 10^2$ | 24 |
| Comparative Example 1 | *E. coli* | $8.9 \times 10^5$ | $1.7 \times 10^4$ | $3.7 \times 10^2$ | 0 |
| | *P. aeruginosa* | $3.3 \times 10^5$ | $9.4 \times 10^3$ | 70 | 1 |
| | *S. aureus* | $3.6 \times 10^5$ | $2.8 \times 10^3$ | $1.8 \times 10^3$ | $2.6 \times 10^2$ |

From the results shown in Table 1, it can be seen that the solutions for contact lenses obtained in Examples 1 and 2 show sufficient disinfecting effects for a contact lens.

The result of the item (2) Adsorption of biguanide derivative (salt) to a material for a contact lens is as shown in Table 2.

Table 2

| Example No. | Adsorbed amount | Adsorbed amount in each immersion (ppm) | | | |
|---|---|---|---|---|---|
| | | 1 | 2 | 3 | 4 |
| 1 | Each adsorbed amount | 2.74 | 2.20 | 0.48 | 0 |
| | Cumulative adsorbed amount | 2.74 | 4.94 | 5.42 | 5.42 |
| 2 | Each adsorbed amount | 1.52 | 1.40 | 0.35 | 0 |
| | Cumulative adsorbed amount | 1.52 | 2.92 | 3.27 | 3.27 |
| Comparative Example 1 | Each adsorbed amount | 9.19 | 4.26 | 4.36 | 1.90 |
| | Cumulative adsorbed amount | 9.19 | 13.45 | 17.81 | 19.71 |
| Comparative Example 2 | Each adsorbed amount | 11.43 | 9.10 | 4.95 | 3.70 |
| | Cumulative adsorbed amount | 11.43 | 20.53 | 25.48 | 29.18 |

From the results shown in Table 2, it can be seen that each amount of the solutions for contact lenses obtained in Examples 1 and 2 and Comparative Example 2, which is adsorbed to the material for a contact lens, is reduced with the increase of the concentration of the phosphoric acid (salt) in each solution for contact lenses in which the material for a contact lens has been immersed.

An amount 1 g of the material for a contact lens was used as a test piece in Examples 1 and 2 and Comparative Example 1. In accordance with each result shown in Table 2, the amount of the biguanide derivative (salt) adsorbed to the material (1 g) was converted into the adsorbed amount of the biguanide derivative (salt) per one contact lens (0.03 g). As a result, the adsorbed amount was about 0.16 ppm in Example 1, or about 0.10 ppm in Example 2. Because the above adsorbed amounts to one contact lens are extremely lower in comparison with the concentration for 50 % growth inhibition of cells of the biguanide derivative (salt), which is about 17 ppm, it can be seen that the solutions for contact lenses obtained in Examples 1 and 2 are excellent in safety for eyes.

Also, it can be seen that the firstly adsorbed amount of the biguanide derivative (salt) is extremely large when the solutions for contact lenses obtained in Comparative Examples 1 and 2 are used, and the amount is 4 to 6 times or so as large as that of the solutions obtained in Examples 1 and 2. Moreover, the adsorption of the biguanide derivative (salt) was attained to saturation when the immersion was repeated 4 times in Exmaples 1 and 2. On the contrary, the adsorption was not attained to saturation even after the immersion was repeated 4 times in Comparative Examples 1 and 2.

Also, in Comparative Example 1, the above immersion was further repeated up to 13 times. As a result, the adsorbed amount was about 2.0 ppm on the average per one immersion, and there was no tendency toward saturation. From the result, it is considered that the material for a contact lens will probably continue to adsorb the biguanide derivative (salt) until the concentration becomes high.

As aforementioned, it can be seen that the solutions for contact lenses obtained in Examples 1 and 2 are hard to adsorb the biguanide derivative (salt) in comparison with the solutions obtained in Comparative Examples 1 and 2.

Accordingly, it can he seen that the solutions for contact lenses obtained in Comparative Examples 1 and 2 are not desirable in the aspect of safety for eyes.

The result of the item (3) Observation of safety by measuring growth inhibition rate is as shown in Table 3.

Table 3

| Example No. | Growth inhibition rate (%) | |
|---|---|---|
| | Added amount | |
| | 100 $\mu\ell$ | 200 $\mu\ell$ |
| 1 | 0 | 0 |
| 2 | 0 | 0 |
| Comparative Example 1 | 43 | 58 |

From the results shown in Table 3, the growth inhibition in the mouse fibroblast L-929 is not observed when the solutions for contact lenses obtained in Examples 1 and 2 are used, therefore, it can be seen that the solutions for contact lenses obtained in Examples 1 and 2 are excellent in safety for eyes.

Also, the growth inhibition rate is extremely high such as 58 % when the added amount of the solution for contact lenses obtained in Comparative Example 1 is 200 $\mu\ell$, therefore, it can be seen that the solution for contact lenses obtained in Comparative Example 1 is bad in safety for eyes.

The result of the item (4) Test for cleaning effect is as shown in Table 4.

Table 4

| Example No. | Result of cleaning |
|---|---|
| 1 | Stains have been completely removed from the contact lens. |
| 2 | Stains have been completely removed from the contact lens. |
| Reference (Saline) | Stains have partly remained in the contact lens. |

From the results shown in Table 4, it can be seen that the solutions for contact lenses obtained in Examples 1 and 2 can completely remove stains from a contact lens.

The result of the item (5) Observation of rinse and storage properties is as shown in Table 5.

Table 5

| Example No. | Existence of microorganisms | |
|---|---|---|
| | Contact lens | Solution for contact lenses |
| 1 | Not observed | Not observed |
| 2 | Not observed | Not observed |
| Reference (Saline) | Observed | Observed |

From the results shown in Table 5, it can be seen that the solutions for contact lenses obtained in Examples 1 and 2 can preserve a contact lens in a sterile state.

In addition to the ingredients used in the Examples, other ingredients can be used in the Examples as set forth in the specification to obtain substantially the same results.

## Claims

1. A solution for contact lenses comprising at least one of a biguanide derivative represented by the general formula (I):

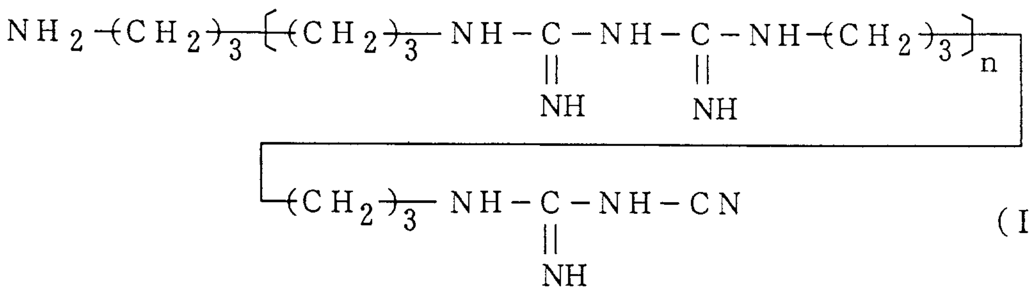

(I)

wherein n is an integer of 1 to 500 and a salt of the biguanide derivative in an effective amount for antimicrobial activity, and
at least one of phosphoric acid and a phosphoric acid salt in an amount of 80 to 250 mM.

2. The solution for contact lenses of Claim 1, wherein said effective amount for antimicrobial activity is 0.1 to 10 ppm.

3. The solution for contact lenses of Claim 1, wherein said phosphoric acid salt is at least one selected from sodium dihydrogen phosphate, disodium hydrogen phosphate, potassium dihydrogen phosphate and dipotassium hydrogen phosphate.

4. The solution for contact lenses of Claim 1, which contains a nonionic surface active agent in an amount of 0.1 to 10 w/v %.

5. The solution for contact lenses of Claim 4, wherein said nonionic surface active agent is a compound having an oxypropylene chain as a main chain and an oxyethylene chain at both ends of the oxypropylene chain.

European Patent Office

# EUROPEAN SEARCH REPORT

Application Number
EP 95 11 4496

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X<br>Y | DE-A-30 07 397 (TITMUS)<br>* claims 1,5; example 1 * | 1-3<br>4,5 | A61L2/18<br>A01N47/44 |
| Y | EP-A-0 079 185 (SMITH & NEPHEW)<br>* page 6, line 12 - page 7, line 19; claims 1,7 * | 4,5 | |
| A | US-A-4 354 952 (RIEDHAMMER)<br>* column 6, line 31 - line 34; claim 1 * | 1-4 | |
| A | FR-A-2 182 962 (SMITH & NEPHEW)<br>* examples 1,5,6,8 * | 1-5 | |
| A | FR-A-2 517 208 (POS) | 1-3 | |
| A | WO-A-94 15649 (POLYMER TECHNOLOGY)<br>* page 6, line 18 - line 22 * | 1-3 | |

TECHNICAL FIELDS SEARCHED (Int.Cl.6)
A61L
A01N

The present search report has been drawn up for all claims

| Place of search | Date of completion of the search | Examiner |
|---|---|---|
| THE HAGUE | 21 December 1995 | Peltre, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)